# EUROPEAN PATENT SPECIFICATION

(11) **EP 0 636 356 B1**
(45) Date of publication and mention of the grant of the patent: **20.12.2000**
(21) Application number: 93420326.6
(22) Date of filing: 27.07.1993
(51) Int. Cl.: A61K 7/06, A61K 7/50, A61K 7/48

(54) **Hair conditioner compositions containing fatty acid ester derivatives of alkanolamines**
Haarkonditioniermittel, die Carbonsäure-Esterderivate von Alkanolaminen enthalten
Compositions de conditionnement des cheveux contenant des dérivés d'esters d'acide d'alkanolamines

(43) Date of publication of application: 01.02.1995
(73) Proprietor: STEPAN COMPANY, Northfield, IL 60093 (US)
(72) Inventor: Sajic, Branko, Chicago, Illinois 60625 (US)
(74) Representative: Guerre, Dominique

(56) References cited:
- EP-A- 0 309 052
- EP-A- 0 367 939
- DE-A- 4 138 630
- GB-A- 2 102 288

## Description

### BACKGROUND OF THE INVENTION

### Field of the Invention

This invention relates to hair conditioning compositions and more specifically it relates to hair conditioning compositions comprising an alkanolamine derivative conditioning compound and a silicone oil conditioning compound.

### Description of the Related Art

Quaternary ammonium compounds have been extensively used as conditioning agents in both rinse-out and leave-on hair conditioners. In addition, long chain alkyl amines and alkanolamines have routinely been incorporated into hair conditioner compositions for use as emulsifiers, suspending or thickening agents. The quarternary compounds mentioned above are well known to provide a conditioning effect on hair by improving detangling, wet combing, dry combing, wet and dry feel and static/fly away. However, quaternary ammonium salts alone are unable to provide sufficient conditioning effects to hair conditioning and especially to hair that is chemically treated, long, or naturally wavy.

For that reason, silicone oil conditioning compounds have been utilized to enhance the performance of hair conditioning compositions. However, silicones are not compatible with all quaternary salts or all conditioner bases. For example, most low molecular weight (low vapor pressure) and high molecular weight (high vapor pressure) silicone compounds, when combined in a conditioner with a di- or tri-long-chain alkyl quaternary ammonium salt, do not yield good conditioning effects on hair. Such conditioners tend to result in an oily feel and poor overall combing properties. See, for example, U.S. Patent No. 4,777,037.

Quaternary ammonium salts have routinely been incorporated into fabric softening compositions. For example, alkanolamine ester quaternary ammonium salts of the general formula I 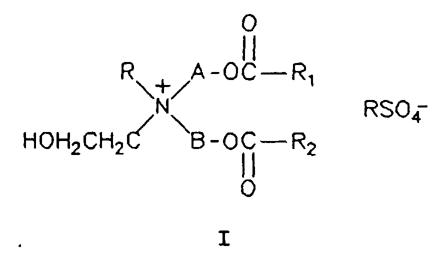 where
R is methyl;
A and B are ethylene;
and R₁ and R₂ are alkyl or alkenyl groups having 14 to 18 carbon atoms;
have been incorporated into various laundry fabric softening compositions. In particular, U.S. Patent No. 3,915,867 discloses the use of N-methyl,N,N-di-(β-C₁₄-C₁₈-acyloxyethyl),N-β-hydroxy ethyl ammonium methylsulfate as the active softening ingredient in a fabric softening composition.

The currently utilized quaternary ammonium salts are frequently made from synthetic sources, are normally not biodegradable, and have a high toxicity towards fish and water plants.

Similar quaternary ammonium compounds have also been incorporated into fabric softeners. See, for example, European Patent application No. EP 295 386 A2; European Patent Application No. EP 370 675 A2; J.A.C.S., 104: 456-61 (1982); and Comun. Jorn. Com. Esp. Deterg. 20: 181-192 (1989).

Silicone oil compounds traditionally have been incorporated into hair conditioning compositions with various quaternary ammonium salts. Exemplary of the quaternary ammonium salts that have been incorporated into silicone containing conditions are: di(hydrogenated) tallow dimethyl ammonium chloride; coco bis(hydroxyethyl) methyl ammonium chloride; trimethyl soyaalkyl ammonium chloride; cocoalkyl trimethyl ammonium chloride; di-C₁₂-C₁₈-alkyl dimethyl ammonium salts; dicocoalkyl dimethyl ammonium chloride; trimethyl tallow ammonium chloride; lauryl trimethyl ammonium chloride; tri-C₈₋₁₀ alkyl methyl ammonium chlorides; and di-C₁₄₋₁₆-alkyl dimethyl ammonium chloride salts.

Representative hair conditioner compositions are disclosed in U.S. Patent No. 4,777,037. That patent discloses hair conditioning compositions which comprise a di-C₁₂₋₁₈-alkyl-di-C₁₋₂-alkyl ammonium salt and a polydimethyl siloxane (a volatile cyclic silicone).

Accordingly, conditioning compositions are desired that employ and a conditioning compound where the conditioning compound is compatible with the silicone as well as prepared from a natural resource, readily and ultimately biodegradable, and only minimally toxic to fish and water plants.

DE-A-41 38 630 discloses hair conditioning compositions containing quaternized fatty acid trialkanolamine ester salts having the following formula III : 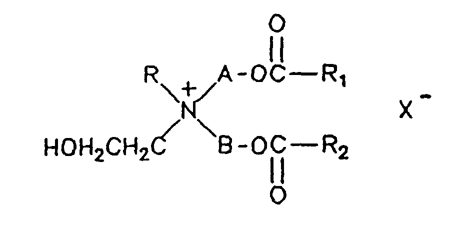 where
R is CH₃,
A and B are the same or different and represent alkyl groups having 2 or 3 carbon atoms,
R₁ and R₂ are the same or different and represent alkyl groups having 8 to 21 carbon atoms, said groups being saturate or having at most one double bond, and
X is Cl, Br, SO₃²⁻, Me or SO₃⁻.

EP-A-0 309 052 discloses fabric softening compositions which may also be used as hair conditioning compositions, which comprise compound of the above-mentioned formula III where :
R is an alkyl group having 1 to 6 carbon atoms,
A and B each represent CH₂-CH₂,
R₁ and R₂ are the same or different and represent alkyl groups having 13 to 21 carbon atoms, and
X is an anion.

Said compositions may optionally comprise up to 15% of a silicone compound.

### SUMMARY OF THE INVENTION

The present invention provides hair conditioner compositions comprising fatty acid ester derivatives of alkanolamines and silicone which impart excellent conditioning properties to hair.

The present invention provides hair conditioners comprising an effective conditioning amount of
an alkanolamine ester of formula II: 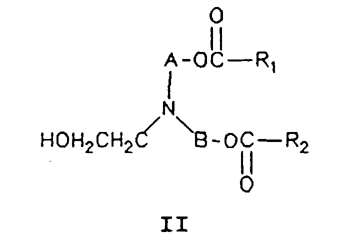 where
   A and B are are the same or different and represent lower alkylene having 1-6 carbon atoms; and
   R₁ and R₂ are the same or different and represent alkyl or alkenyl groups having 8 to 40 carbon atoms;
and a silicone oil conditioning compound.

The present invention further provides hair conditioning compositions comprising a silicone oil conditioning compound and an alkanolamine derivative of Formula II and an alkanolamine ester quarternary ammonium salt of formula III: 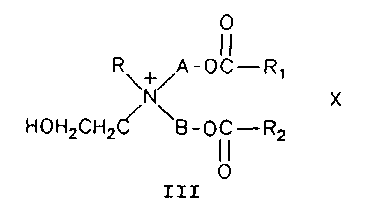 where
R is lower alkyl having 1-6 carbon atoms;
A and B are the same or different and represent lower alkylene having 1-6 carbon atoms; and
R₁ and R₂ are the same or different and represent alkyl or alkenyl groups having 8 to 40 carbon atoms; and
X is Cl⁻, Br⁻, I⁻, RSO₃⁻, or RSO₄⁻.

It has been surprisingly discovered that the alkanolamine derivatives of formulas II and III are capable of functioning efficiently in combination with silicone conditioning agents of various vapor pressures and molecular weights.

### DETAILED DESCRIPTION OF THE INVENTION

It has been unexpectedly discovered that when hair conditioning compositions are prepared to contain a silicone oil conditioning compound and a fatty acid ester derivative of an alkanolamine, the resultant hair conditioning compositions provide excellent conditioning effects on hair.

Thus, the present invention provides hair conditioners comprising an effective conditioning amount of
an alkanolamine ester of formula II: 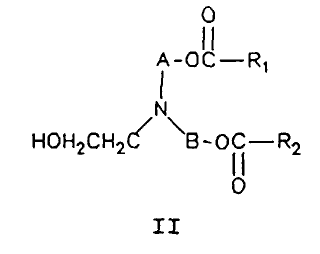 where
   A and B are are the same or different and represent lower alkylene having 1-6 carbon atoms; and
   R₁ and R₂ are the same or different and represent alkyl or alkenyl groups having 8 to 40 carbon atoms;
and a silicone oil conditioning compound

In addition, the present invention encompasses hair conditioning compositions comprising effective conditioning amounts of a compound of formula II, a compound of formula III and a silicone oil conditioning compound.

In the hair conditioning compositions of the present invention, these alkanolamine derivatives function in tandem with silicone oil conditioning compounds to provide conditioning effects on hair. The alkanolamine derivatives of formulas II and III were surprisingly discovered to be capable of functioning efficiently in combination with silicone conditioning agents of various vapor pressures and molecular weights. These alkanolamine derivatives provide excellent conditioner compositions with silicones of a wide range of molecular weights and vapor pressures.

The hair conditioning compositions prepared to contain both an alkanolamine derivative of formula II and a silicone conditioning compound and optionally an alkanolamine derivative of formula III were also unexpectedly found to exhibit conditioning properties greater than the aggregate of the conditioning properties of each individual component. In addition, the compositions of the present invention are prepared using alkanolamine derivatives that demonstrate minimal fish and water plant toxicity, are readily and ultimately biodegradable and are prepared from simple natural resources.

The present invention relates to hair conditioning compositions which contain water and an alkanolamine derivative conditioning agent and a silicone oil conditioning compound. This alkanolamine derivative has the chemical formula II shown above. The invention also relates to hair conditioning compositions which contain water and a combination of a silicone and an alkanolamine derivative of formula II and III. The compositions of this invention are useful both as water dispersions or aqueous emulsions preferably stable emulsions in which water comprises the external phase.

Those skilled in the art will recognize a variety of synthetic methodologies for preparing the alkanolamine derivatives of formulas II and III. Exemplary of suitable preparative methods for synthesizing an alkanolamine ester of formula II is an acid catalyzed esterification of a fatty acid with an alkanol. In addition, such an alkanolamine ester may be prepared by a base-catalyzed transesterification of a methyl ester in the presence of an alkanolamine.

The resulting alkanolamine esters may be quaternized using any suitable quaternizing agent, such as for example, dimethyl sulfate.

A synthesis of compounds of these classes is found in U.S. Patent No. 3,915,867.

The silicone compounds which may be used in the hair conditioning compositions of the present invention are well known and include those that have been generally taught to be useful in a variety of emulsions. The silicones useful for incorporation into the conditioning compositions of the invention are silicone fluids as well as silicone gums. Such silicones will have viscosities ranging from a few hundred centipoise to about 1,0000,000 centipoise. A blend of high and low silicones can be utilized in order to obtain the desired conditioning effect and to facilitate incorporation into a hair conditioner product.

Among the silicone compounds suitable for incorporation into conditioning compositions of the invention are dimethicones and cyclomethicones which may be represented by the formula: 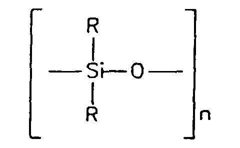 wherein R is a 1 to 3 carbon alkyl group, n is a integer from 3 to 10, preferably from 3 to 7, and the unsatisfied valences on the oxygen and silicon atoms at the ends of the chain may be joined to one another to form a cyclic structure. Suitable silicone compounds are, for example, U.C.C. Y-7207, gold by Union Carbide Corporation in which each R is methyl and which typically comprises by weight 99.4% tetramer, 0.6% trimer and traces of the pentamer and hexamer; and SWS-03314, sold by SWS Silicones, a Division of Stauffer Chemical Company, in which R is methyl and which is substantially all tetramer; and Dow Corning 344 fluid, sold by Dow Corning, Inc., in which R is methyl and which typically comprises by weight about 88% tetramer, about 11.8% pentamer and traces of trimer and hexamer. Typical vapor pressures of silicones are shown in the table below. These vapor pressures were determined using Dow Corning 344 fluid at various temperatures.

| Temperature | Vapor Pressure, mm Hg |
|---|---|
| 2oC | 1 |
| 64°C | 10 |
| 77°C | 20 |
| 92°C | 40 |
| 101°C | 60 |
| 114°C | 100 |
| 155°C | 400 |
| 178°C | 760 |

Such silicones are said by one manufacturer to be useful in various cosmetic compositions such as antiperspirants, deodorants, hair sprays, hair coloring and hair grooming products, and because of their low viscosity and surface tension provide a light silky feel on hair and skin. These silicones, are also reported to be non-greasy but to provide subtle lubrication.

Also suitable for use in the invention are amodimethicones of the formula: 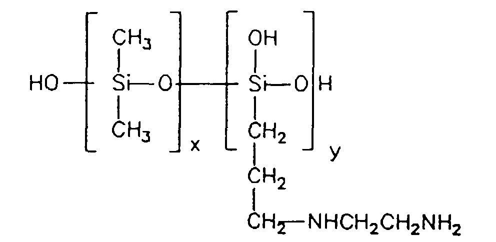
where x is an integer at least equal to 4, and y is an integer at least equal to 4.
Such silicone compounds may be synthesized to have molecular weights and vapor pressures that range from low to high. In addition, such amodimethicones may be quaternized to yield cationic amine functional polymer emulsions. Representative emulsions are available from Dow Corning and include 929 cationic emulsion, Silicone Q2-7224, and Silicone DC X2-8939.

The preferred compositions of the invention typically include water, an alkanolamine derivative of formula II and an alkanolamine derivative of formula III. Water typically constitutes at least about 70 weight percent of the weight of the conditioning composition, and more preferably about 80 weight percent.

The silicones may be present in the hair conditioning compositions of this invention in amounts of from about 0.5 to about 15 weight percent of the composition. Preferably, these silicones are present at from about 1 to about 10 percent by weight of the conditioning composition. Particularly preferred amounts of silicone in the compositions of the invention are from about 1.0 to about 6 percent of the composition.

The alkanolamine derivative conditioning agent of formula II is preferably present at from about 0.5 to about 20 percent by weight of the composition as an active ingredient. More preferably, said alkanolamine derivative conditioning agent is present at from about 1 to about 5 weight percent, as an active ingredient. In particularly preferred compositions, alkanolamine derivative is present at from about 1-3 percent of the composition.

In certain compositions according to the invention, the alkanolamine derivative is present as a mixture of an alkanolamine ester and alkanolamine ester quaternary ammonium salt. In such compositions, the combined amount of the alkanolamine ester and alkanolamine ester quaternary ammonium salt ranges from about 0.1% to about 20% of the conditioner compositon. In these compositons, the ratio of alkanolamine ester to alkanolamine ester quaternary ammonium salt ranges from about 1:99 to about 99:1. In more preferred compositons, the ratio of alkanolamine ester to alkanolamine ester quaternary ammonium salt ranges between about 1:75 to about 1:10.

In certain embodiments of the invention, the alkanolamine derivative is introduced into the composition as an about 50-99% mixture of the alkanolamine derivative in alcohol or glycol. In certain preferred embodiments, the alkanolamine derivative conditioning compound is introduced as an about 70-80% mixture of the alkanolamine derivative in the alcohol or glycol. Particularly preferred embodiments of the invention are prepared using a mixture of the alkanolamine derivative in propylene glycol, ethanol or isopropanol.

Preferred alkanolamine derivatives of formula III according to the invention are N-methyl, N,N-di(β-partially hydrogenated talloyloxyethyl), N-β-hydroxyethyl ammonium methyl sulfate; and N-Methyl, N,N-di(β-partially hydrogenated palmitoyloxy ethyl), N-β-hydroxy ethyl ammonium methyl sulfate.

It is noted that the long aliphatic chains of the the quaternary ammonium conditioning agents, designated by R₁ and R₂ in formulas II and III above, need not be solely or primarily of one chain length; i.e., the long chain need not be cetyl, myristyl, lauryl or stearyl. Rather, conditioning agents having long aliphatic chains R₁ and R₂ containing a mixture of lengths can be used. Such conditioning agents are conveniently prepared from naturally occurring materials, such as tallow, coconut oil, soya oil and the like, or from synthetically produced mixtures.

The compositions of this invention containing only water, silicone and the alkanolamine derivative are milky-white, relatively viscous dispersions. Those compositions are stable to phase separation at a temperatures of about 0° to about 45°C for at least 30 days after their preparation, and are typically stable to phase separation indefinitely at temperatures of from about 20 to 25°C.

The compositions of this invention can also be in the form of emulsions that contain additional amounts of hydrophilic and/or hydrophobic emulsifiers. Emulsions containing additional emulsifier materials are particularly preferred. It is preferred that those emulsions be stable to phase separation at a temperature of about 45°C for a period of about 30 days after their preparation. The emulsions are more preferably stable to phase separation at temperature normally found in commercial product storage and shipping for periods of six months or more.

The compositions of the invention may also contain thickeners or emulsifiers. The thickeners and emulsifiers may be long chain fatty alcohols having from about 11 to about 22 carbon atoms. These alcohols can be used alone, or in admixture with each other. When included in the compositions, the alcohol is preferably present at from about 0.5 to about 10 weight percent of the composition, and more preferably at from about 2 to about 5 weight percent.

Lauryl alcohol, oleyl alcohol, myristyl alcohol, cetyl alcohol, stearyl alcohol, and the like, and mixtures thereof are contemplated herein. In addition, mixtures of natural or synthetic fatty alcohols having fatty chain lengths of from about 11 to about 22 carbons are also useful. Several such mixtures are available commercially, and are exemplified by the material containing a mixture of synthetic alcohols with 12 to 15 carbons in the alkyl chain sold under the trademark NEODOL 25 by Shell Chemical Company, and the material containing a mixture of synthetic alcohols with chain lengths of 12 to 18 carbons sold under the trademark ALFOL 1216 Alcohol by Conoco Chemicals.

Fatty alcohols of the above discussed carbon chain lengths which are ethoxylated to contain an average of one or two moles of ethylene oxide per mole of fatty alcohol can be used in place of the fatty alcohols themselves. Examples of such useful ethoxylated fatty acids include ethylene glycol cetyl ether, polyoxyethylene (2) stearyl ether, and the like; the exemplary compounds having CTFA Dictionary names of Ceteth-1 and Steareth-2, respectively.

The thickeners and emulsifiers suitable for use in the invention may also be compounds such as, for example, hydroxyethylcellulose (available under the tradename Natrosol® from Aqualon), hydroxypropyl methylcellulose (available under the tradename Methocel® from Dow Chemical), xanthan gum (available under the tradenames Keltrol® and Kelzan® from Kelco), and N,N-dimethyl, N-stearyl amine (available under the tradename Lexamine® S-13 from Inolex).

A tertiary amidoamine can also be present in the hair conditioning compositions of this invention, and is present in particularly preferred compositions at a concentration of from about 0.1 to about 2 weight percent of the composition, and more preferably at from about 0.25 to about 1 weight percent.

The tertiary amidoamines useful herein have structures conforming to the formula R¹-C(=O)-N-H-R²-N(R³)₂ wherein R¹ is a fatty chain having about 11 to about 17 carbon atoms, R² is an alkylene group having 2 or 3 carbon atoms and each R³ is ethyl or methyl. Exemplary, useful, tertiary amidoamines include dimethylaminopropyl stearmide, diethylaminoethyl stearamide and dimethylaminopropyl myristamide. The R¹ group of the tertiary amidoamines can also be prepared from coconut, soya and tallow fatty acids, or the like.

The hair conditioning compositions of this invention suitably are near neutral to slightly acidic in pH value. Thus, the hair conditioners of this invention preferably have pH values of from about 3 to about 8, and more preferably from about 3.5 to about 6.0.

Ingredients in addition to water and the previously discussed ingredients can also be present, in the composition of this invention. These additional ingredients include, but are not limited to, polyhydric alcohols, such as propylene glycol or glycerin; hydroxyethylated fatty alcohols having from 12 to 18 carbon atoms in the fatty chain and an average of about 15 to about 30 moles of ethylene oxide added per mole of alcohol; opacifiers, which are inorganic salts such as, for example, sodium chloride, potassium chloride, ammonium chloride, or magnesium sulfate; or perfumes, colorants, preservatives and the like. Suitable hydroxyethylated fatty alcohols include the previously described fatty alcohols having from 11 to 18 carbon atoms which contain the desired amount of hydroxyethylation such as polyoxyethylene (20) cetyl ether, polyoxyethylene (30) stearyl ether, polyoxyethylene (15) lauryl ether, the polyoxyethylene glycol ether of synethic fatty alcohols having about 11 to 15 carbons in the fatty chain and an average of 20 moles of ethylene oxide per mole of alcohol, and the polyethylene glycol ether of fatty alcohols containing primarily cetyl and stearyl alcohol and an average of 20 moles of ethylene oxide per mole of alcohol. These exemplary hydroxyethylated fatty alcohols are given the following *CTFA Dictionary* names, respectively: Ceteth-20, Steareth-30, Laureth-15, Pareth-15-20 and Ceteareth-20. Conditioners according to the invention may optionally include presevatives such as, for example, 1,3,5,5-tetramethyl hydantoin (DMDM hydantoin).

The compositions of the invention provided excellent results in the areas of detangling, wet- and dry-combability, wet and dry feel, static/flyaway, sheen, body, etc. These conditioning properties were demonstrated with conditioner compositions incorporating a variety of silicone compounds having a wide range of molecular weights and vapor pressures. These results were demonstrated in salon tests and in laboratory tests on swatches of hair. As pointed out above, the hair conditioning compositions prepared to contain both an alkanolamine derivative of formula II and a silicone conditioning compound were unexpectedly found to exhibit conditioning properties greater than the aggregate of the conditioning properties of each individual component.

The conditioning compositions of the present invention are readily manufactured using any conventional emulsification process. The manufacture of the conditioners may be effected using either single-phase hot processes or multi-phase processes.

### Example 1

### Preparation of N-methyl, N,N-di(β-partially hydrogenated talloyloxyethyl), N-β-hydroxy ethyl ammonium methyl sulfate

### 1. N,N-di(β-partially hydrogenated talloyloxyethyl), N-β-hydroxy ethylamine

To a suitable reaction flask equipped with a stirrer, nitrogen inlet and thermometer was added 81.4 g of partially hydrogenated tallow fatty acid. The atmosphere in the flask was purged with nitrogen, after which phosphoric acid (H₃PO₄, 0.12g) and triethanolamine (TEA, 24.4g) were added. The reaction flask was heated at about 155-160°C while removing water until the reaction was complete.

The resulting N,N-di(β-partially hydrogenated talloyloxyethyl), N-β-hydroxy ethylamine may be incorporated into conditioner compositions or may be quaternized as described below. If it is used as the free amine, it can be added neat or as a mixture of the amine in an alcohol or glycol.

### 2. Quaternization with dimethyl sulfate

To 100g of the diester amine product prepared have in part 1 was added 19.1g of dimethyl sulfate and the mixture heated for about 2 hours at 97°C. A viscous diester quaternary ammonium dimethyl sulfate product was obtained. Prior to incorporation into a conditioner composition, the diester quaternary ammonium methyl sulfate product was mixed with isopropanol, ethanol or propylene glycol to yield an about 50-99% mixture of the quaternary ammonium methyl sulfate in isopropanol, ethanol, or propylene glycol. The diester quaternary ammonium methyl sulfate product prepared according to this procedure may also contain triethanolamine and/or diester amine.

### Example 2

### Preparation of N-Methyl, N,N-di(β-partially hydrogenated palmitoyloxy ethyl), N-β-hydroxyethyl ammonium methyl sulfate

### 1. N,N-di(β-partially hydrogenated palmitoyloxy ethyl), N-β-hydroxy ethylamine

A 4-neck, 1 liter reaction flash fitted with a stirrer, thermometer, nitrogen inlet, and a vacuum distillation assembly was charged with 1.78 moles of partially hydrogenated palm fatty acid methyl ester, 133.2g (0.893 moles) of TEA and 8g of a 25% solution of sodium methoxide. The mixture was stirred and heated to 91-105°C under vacuum (about 28 inches of Hg) and nitrogen flow (about 5° cc/min) for about 1.75 hours. 579g of resulting amine ester were obtained.

The resulting N,N-di(β-partially hydrogenated palmitoyloxy ethyl), N-β-hydroxy ethylamine may be incorporated into conditioner compositions or may be quaternized as described below. If it is used as the free amine, it can be added neat or as a mixture of the amine in an alcohol or glycol.

### 2. Quaternization with dimethyl sulfate

The diester amine obtained in part 1 of Example 2 above was quaternized with dimethyl sulfate using substantially the same procedure as set forth in part 2 of Example 1. Prior to use in a conditioner composition, the diester quaternary ammonium methyl sulfate product was mixed with isopropanol, ethanol or propylene glycol to yield an about 50-95% mixture of diester quaternary ammonium methyl sulfate in isopropanol, ethanol, or propylene glycol. The diester quaternary ammonium methyl sulfate product prepared according to this procedure may also contain triethanolamine and/or diester amine.

### Example 3

### Preparation of a Hair Conditioner Composition

To a suitable vessel equipped with stirring, heating, and cooling capabilities was added 426g of deionized water, 2.5g of N,N-di(β-partially hydrogenated palmitoyloxy ethyl), N-β-hydroxyethyl amine, and 11.0g of a 70% mixture of N,N-di(β-partially hydrogenated palmitoyloxy ethyl), N-β-hydroxyethyl ammonium methyl sulfate in propylene glycol. This mixture was stirred and heated to 70-75°C. At 70-75°C, 15g of cetyl alcohol were added and the mixture emulsified for 20-30 minutes at 70-75°C. The mixture was then cooled about 50°C at which time 6.5g of a 20 percent aqueous potassium chloride (KCl) solution was added. This mixture was then stirred well and 10g of Silicone DC 345, 28.5g of Silicone X2-8939, and 1.00g of DMDM hydantoin were added. The composition, 3A, was stirred until homogeneous; and the pH was checked and adjusted if necessary to between 4.0 and 4.5 with 50% aqueous citric acid or 50 percent aqueous NaOH.

Conditioner compositions 3B, 3C, 3D, 3E, 3F, and 3G were prepared using essentially the same procedure set forth above for preparing composition 3A. Table I below shows the amount of each component in compositions 3A-3E. Each of these compositions resulted in excellent conditioning of hair as demonstrated in both salon and laboratory settings.

**Table I**

| Component¹ | 3A | 3B | 3C | 3D | 3E |
|---|---|---|---|---|---|
| Deionized water | 85.09 | 85.79 | 93.50 | 93.80 | 85.09 |
| hydroxyethylcellulose | -- | -- | -- | 0.70 | 0.70 |
| palm ester amine² | 0.50 | 2.00 | 2.00 | 2.00 | 2.00 |
| palm ester quaternary ammonium salt³ | 2.20 | -- | -- | -- | -- |
| cetyl alcohol | 3.00 | 3.00 | 3.00 | 2.00 | 3.00 |
| 20% aqueous KCl | 1.30 | 1.30 | 1.30 | 1.30 | 1.30 |
| Silicone DC 345 | 2.00 | 2.00 | -- | -- | 2.00 |
| Silicone DC X2-8939 | 5.71 | 5.71 | -- | -- | 5.71 |
| 50% aq. NaOH | Q.S. | Q.S. | Q.S. | Q.S. | Q.S. |
| 50% aq. citric acid | Q.S. | Q.S. | Q.S. | Q.S. | Q.S. |
| DMDM hydantoin | 0.20 | 0.20 | 0.20 | 0.20 | 0.20 |

| | | | | | |
|---|---|---|---|---|---|
| 1 component amounts are expressed as percent of final composition | | | | | |
| 2 N,N-di(β-partially hydrogenated palmitoyloxy ethyl), N-β-hydroxyethyl amine (neat) | | | | | |
| 3 N-methyl, N,N-di(β-partially hydrogenated palmitoyloxy ethyl), N-β-hydroxy ethyl ammonium methyl sulfate (75% in propylene glycol) | | | | | |

## Claims

1. A hair conditioning composition comprising an effective conditioning amount of :
- an alkanolamine ester of formula II : 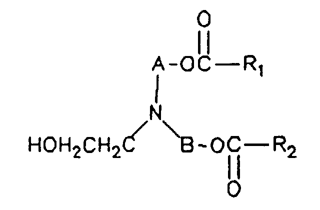 where
A and B are the same or different and represent lower alkylene having 1-6 carbon atoms, and
R₁ and R₂ are the same or different and represent alkyl or alkenyl groups having 8 to 40 carbon atoms;
and
- a silicone oil conditioning compound.

2. A conditioning composition according to claim 1, further comprising an effective conditioning amount of an alkanolamine ester quaternary ammonium salt of formula III : 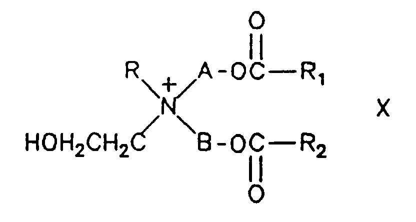 where
R is lower alkyl having 1-6 carbon atoms;
A and B are the same or different and represent lower alkylene having 1-6 carbon atoms, and
R₁ and R₂ are the same or different and represent alkyl or alkenyl groups having 8 to 40 carbon atoms, and
X is Cl, Br, I, RSO₃, or RSO₄,

3. A conditioning composition according to claim 1, wherein the amount of the alkanolamine ester in the composition is 0.5 % to 20 % by weight of the composition.

4. A conditioning composition according to claim 3, wherein the amount of the alkanolamine ester in the composition is 1 % to 5 % by weight of the composition.

5. A conditioning composition according to claim 2, wherein the combined amount of the alkanolamine ester and the alkanolamine ester quaternary ammonium salt in the composition is 0.1 % to 20 % by weight of the composition.

6. A conditioning composition according to claim 1 or 2, wherein the amount of the silicone conditioning compound is about 0.5 % to about 15 % by weight of the composition.

7. A conditioning composition according to claim 1 or 2, wherein the amount of the silicone conditioning compound is about 1 % to about 10 % by weight of the composition.

8. A composition according to claim 1, wherein the alkanolamine ester is N,N-di(β-partially hydrogenated palmitoyloxy ethyl), N-β-hydroxyethyl amine.

9. A composition according to claim 2, wherein the alkanolamine ester quaternary ammonium salt is N-methyl, N,N-di(β-partially hydrogenated palmitoyloxy ethyl), N-β-hydroxyethyl ammonium methyl sulfate.

10. A composition according to claim 1, wherein the silicone conditioning compound is selected from the group consisting of amodimethicones, dimethicones, cyclomethicones, and mixtures thereof.

11. A dispersion comprising a composition according to any one of claims 1 to 10.

12. An emulsion comprising a composition according to any one of claims 1 to 10, and an emulsifier.

## Patentansprüche

1. Haarkonditionierzusammensetzung mit einer wirksamen konditionierenden Menge an
- einem Alkanolaminester der Formel II 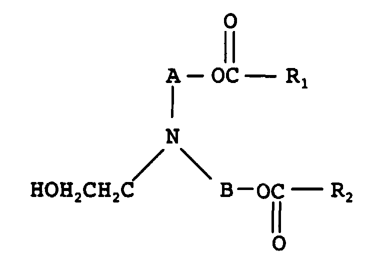 in der
A und B gleich oder unterschiedlich sind und für niedere Alkylene mit 1 bis 6 Kohlenstoffatomen stehen, und
R₁ und R₂ gleich oder unterschiedlich sind und für Alkyl- oder Alkylengruppen mit 8 bis 40 Kohlenstoffatomen stehen, und
- einer Siliconöl-Konditionierkomponente.

2. Konditionierzusammensetzung nach Anspruch 1, die ferner eine wirksame konditionierende Menge eines quarternären Ammoniumsalzes eines Alkanolaminesters der Formel III enthält 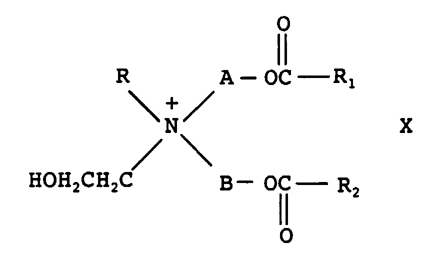 in der
R ein niederes Alkyl mit 1 bis 6 Kohlenstoffatomen ist;
A und B gleich oder unterschiedlich sind und für ein niederes Alkylen mit 1 bis 6 Kohlenstoffatomen stehen, und
R₁ und R₂ gleich oder unterschiedlich sind und für eine Alkyl- oder Alkylengruppe mit 8 bis 40 Kohlenstoffatomen stehen, und
X ist Cl, Br, J, RSO₃ oder RSO₄.

3. Konditionierzusammensetzung nach Anspruch 1, dadurch gekennzeichnet, daß der Gehalt an Alkanolaminester in der Zusammensetzung 0,5 bis 20 Gew.-% der Zusammensetzung beträgt.

4. Konditionierzusammensetzung nach Anspruch 3, dadurch gekennzeichnet, daß der Gehalt an Alkanolaminester in der Zusammensetzung 1 bis 5 Gew.-% der Zusammensetzung beträgt.

5. Konditionierzusammensetzung nach Anspruch 2, dadurch gekennzeichnet, daß die vereinigte Menge an Alkanolaminester und dem quarternären Ammoniumsalz des Alkanolaminesters in der Zusammensetzung 0,1 bis 20 Gew.-% der Zusammensetzung beträgt.

6. Konditionierzusammensetzung nach Anspruch 1 oder 2, dadurch gekennzeichnet, daß die Menge an Silicon-Konditionierkomponente etwa 0,5 bis 15 Gew.-% der Zusammensetzung beträgt.

7. Konditionierzusammensetzung nach Anspruch 1 oder 2, dadurch gekennzeichnet, daß die Menge an Silicon-Konditionierkomponente etwa 1 bis 10 Gew.-% der Zusammensetzung beträgt.

8. Zusammensetzung nach Anspruch 1, bei der der Alkanolaminester N,N-di(β-teilweise hydriertes Palmitoyloxyethyl), N-β-hydroxyethylamin ist.

9. Zusammensetzung nach Anspruch 2, bei der das quarternäre Ammoniumsalz des Alkanolaminesters N-Methyl,N,N-di(β-teilweise hydriertes palmitoyloxyethyl),N-β-hydroxyethylammoniummethylsulfat ist.

10. Zusammensetzung nach Anspruch 1, bei der die Silicon-Konditionierungskomponente ausgewählt ist aus der Gruppe bestehend aus Amodimethiconen, Dimethiconen, Cyclomethiconen und Mischungen davon.

11. Dispersion enthaltend eine Zusammensetzung nach einem der Ansprüche 1 bis 10.

12. Emulsion enthaltend eine Zusammensetzung nach einem der Ansprüche 1 bis 10 und einen Emulgator.

## Revendications

1. Composition de conditionnement des cheveux contenant une quantité à effet conditionneur :
- d'un ester d'alcanolamine de formule II : 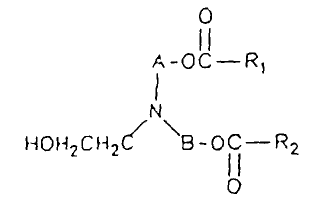 dans laquelle
A et B sont identiques ou différents, et représentent un alkylène inférieur comportant 1 à 6 atomes de carbone, et
R₁ et R₂ sont identiques ou différents, et représentent des groupes alkyle ou alcényle comportant 8 à 40 atomes de carbone;
et
- d'un composé conditionneur du type huile de silicone.

2. Composition de conditionnement selon la revendication 1, comprenant également une quantité à effet conditionneur d'un sel d'ammonium quaternaire d'un ester d'alcanolamine, de formule III : 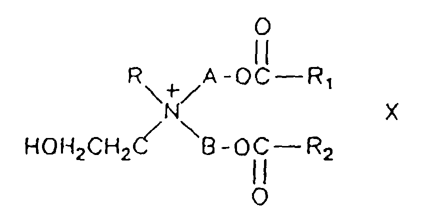 dans laquelle
R est un alkyle inférieur comportant 1 à 6 atomes de carbone;
A et B sont identiques ou différents, et représentent un alkylène inférieur comportant 1 à 6 atomes de carbone, et
R₁ et R₂ sont identiques ou différents, et représentent des groupes alkyle ou alcényle comportant 8 à 40 atomes de carbone, et
X est Cl, Br, I, RSO₃, ou RSO₄.

3. Composition de conditionnement selon la revendication 1, dans laquelle la quantité d'ester d'alcanolamine présente dans la composition est comprise entre 0,5 % et 20 % en poids de la composition.

4. Composition de conditionnement selon la revendication 3, dans laquelle la quantité d'ester d'alcanolamine présente dans la composition est comprise entre 1 % et 5 % en poids de la composition.

5. Composition de conditionnement selon la revendication 2, dans laquelle la quantité combinée d'ester d'alcanolamine et de sel d'ammonium quaternaire de l'ester d'alcanolamine, dans la composition, est comprise entre 0,1 % et 20 % en poids de la composition.

6. Composition de conditionnement selon la revendication 1 ou 2, dans laquelle la quantité du composé conditionneur au silicone est comprise entre environ 0,5 % et environ 15 % en poids de la composition.

7. Composition de conditionnement selon la revendication 1 ou 2, dans laquelle la quantité du composé conditionneur au silicone est comprise entre environ 1 % et environ 10 % en poids de la composition.

8. Composition selon la revendication 1, dans laquelle l'ester d'alcanolamine est la N-β-hydroxyéthylamine du N,N-di(palmitoyloxyéthyle partiellement hydrogéné en β).

9. Composition selon la revendication 2, dans laquelle te sel d'ammonium quaternaire de l'ester d'alcanolamine est le méthylsulfate de N-méthyl N,N-di(palmitoyloxyéthyle partiellement hydrogéné en β) N-β-hydroxyéthyl ammonium.

10. Composition selon la revendication 1, dans laquelle le composé conditionneur au silicone est choisi dans le groupe constitué des amodiméthicones, des diméthicones, des cyclométhicones, et des mélanges de celle-ci.

11. Dispersion comprenant une composition selon l'une quelconque des revendications 1 à 10.

12. Émulsion comprenant une composition selon l'une quelconque des revendications 1 à 10, et un émulsifiant.
